# NEUE EUROPÄISCHE PATENTSCHRIFT

(11) **EP 1 027 440 B2**
(45) Veröffentlichungstag und Bekanntmachung des Hinweises auf die Entscheidung über den Einspruch: **14.09.2011**
(45) Hinweis auf die Patenterteilung: 09.04.2008
(21) Anmeldenummer: 98959767.9
(22) Anmeldetag: 27.10.1998
(51) Int. Cl.: C12N 15/18, C07K 14/475, C07K 16/22, C12N 5/10, A61K 38/22, A61K 48/00, G01N 33/53, C12Q 1/68

(54) **INHIBITOR-PROTEIN DES WNT-SIGNALWEGS**
INHIBITOR PROTEIN OF THE WNT SIGNAL PATHWAY
PROTEINE INHIBITRICE DE LA VOIE DE SIGNALISATION WNT

(30) Priorität: 27.10.1997 DE 19747418
(43) Veröffentlichungstag der Anmeldung: 16.08.2000
(60) Teilanmeldung: 08002377.3
(73) Patentinhaber: Deutsches Krebsforschungszentrum Stiftung des Öffentlichen Rechts, 69120 Heidelberg (DE)
(72) Erfinder: NIEHRS, Christof, D-69117 Heidelberg (DE); GLINKA, Andrei, D-69126 Heidelberg (DE)
(74) Vertreter: Schüssler, Andrea
(86) Internationale Anmeldenummer: PCT/DE1998/003155
(87) Internationale Veröffentlichungsnummer: WO 1999/022000

(56) Entgegenhaltungen:
- WO-A-98/46755
- SAWADA K ET AL: "Characterization of terminally differentiated cell state by categorizing cDNA clones derived from chicken lens fibers." INT J DEV BIOL, JUN 1996, 40 (3) P531-5, XP002096086 SPAIN -& EMVRT DATABASE Accession number D26311 29-JUL-1994 (Rel. 40, Created) Sawada K XP002096089
- GLINKA A ET AL: "Head induction by simultaneous repression of Bmp and Wnt signalling in Xenopus." NATURE, OCT 2 1997, 389 (6650) P517-9, XP002096087 ENGLAND
- GLINKA, ANDREI ET AL: "Dickkopf-1 is a member of a new family of secreted proteins and functions in head induction" NATURE (LONDON) (1998), 391(6665), 357-362 CODEN: NATUAS;ISSN: 0028-0836, XP002096088
- FINCH P.W. ET AL: 'PURIFICATION AND MOLECULAR CLONING OF A SECRETED, FRI' PROCEEDINGS OF THE NAT. ACADEMY OF SCIENCES OF THE US (PNAS) Bd. 94, 24 Juni 1997, NATIONAL ACADEMY OF SCIENCE, US, ISSN 0027-8424 Seiten 6770 - 6775

## Beschreibung

Die vorliegende Erfindung betrifft ein Inhibitor-Protein des wnt-Signalwegs, eine ein solches Protein kodierende DNA und ein Verfahren zur Herstellung eines solchen Proteins. Ferner betrifft die Erfindung die Verwendung der DNA und des Proteins sowie gegen das Protein gerichtete Antikörper.

Der wnt-Signalweg spielt eine wichtige Rolle in der Regulation der Zellproliferation und -Differenzierung während der Embryonal-Entwicklung von Drosophila, Xenopus laevis und der Maus. Der wnt-Signalweg umfaßt die Kombination von sekretorischen Glykoproteinen, die durch wnt-Gene, z.B. Xwnt-8, kodiert sind, und wnt-Rezeptoren, an die die Glykoproteine binden. Ferner ist der wnt-Signalweg beim Menschen kausal im Colon- und Mammakarzinom sowie dem Melanom impliziert (vgl. Peifer, M., Science 275, (1997), 1752-1753). Inhibitoren des wnt-Signalwegs könnten daher eine Möglichkeit darstellen, therapeutisch bei Tumorerkrankungen eingreifen zu können.

Die US-Prioritätspatentmeldungen 08/843,704 und 08/842,898, die mit der PCT Veroffentlichung WO 98 46755 weitgehend übereinstimmen, beschreiben zwar bestimmte CRSP-Proteine (dkk3 = hCRSP-1 = T59), allerdings ohne irgendeine Funktion.

Der vorliegenden Erfindung liegt somit die Aufgabe zugrunde, ein Mittel bereitzustellen, mit dem der wnt-Signalweg inhibiert werden kann.

Erfindungsgemäß wird dies durch die Gegenstände in den Patentansprüchen erreicht.

Gegenstand der vorliegenden Erfindung ist somit ein Inhibitor-Protein des wnt-Signalwegs, gemäß Anspruch 1.

Die vorliegende Erfindung beruht auf der Erkenntnis des Anmelders, daß in Tieren, besonders Säugetieren, ganz besonders dem Menschen, ein Protein existiert, das den wnt-Signalweg inhibiert. Der Anmelder hat gefunden, daß die

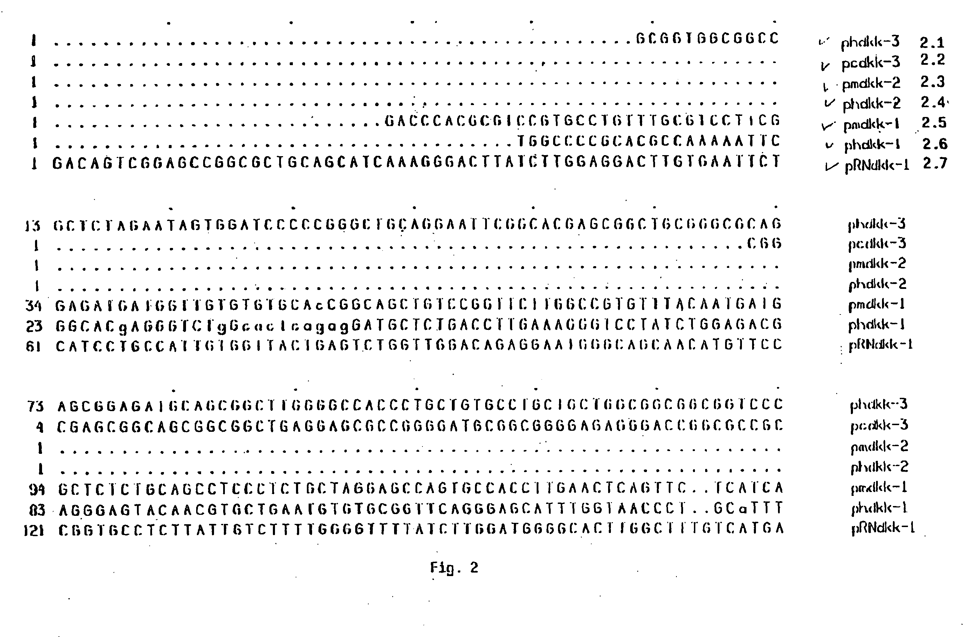

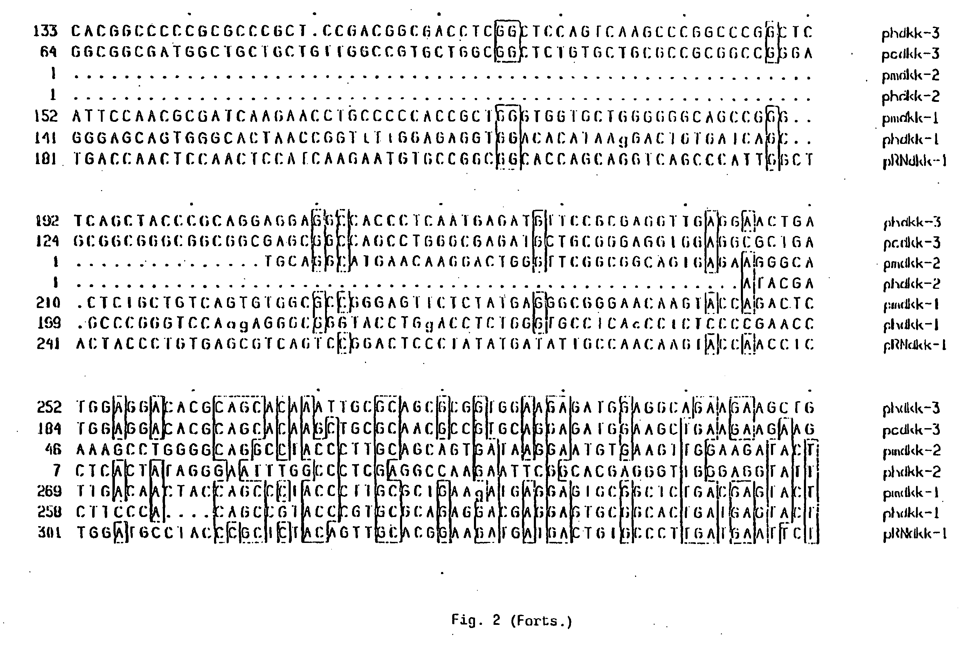

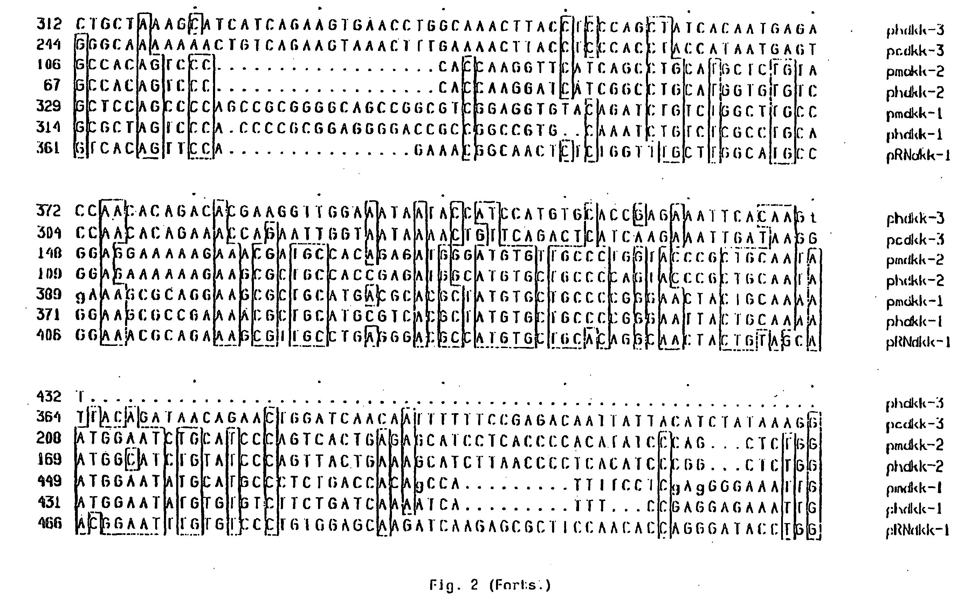

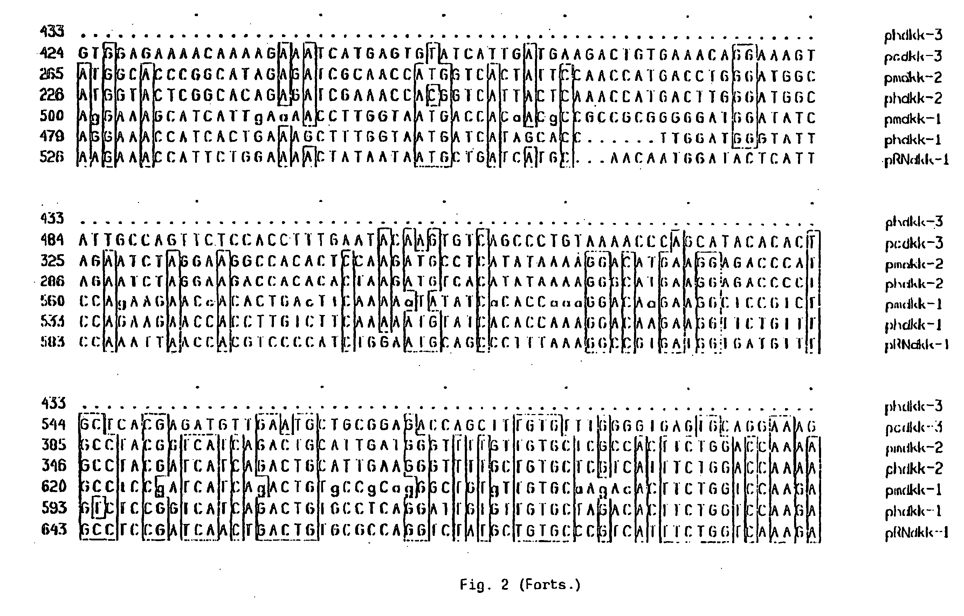

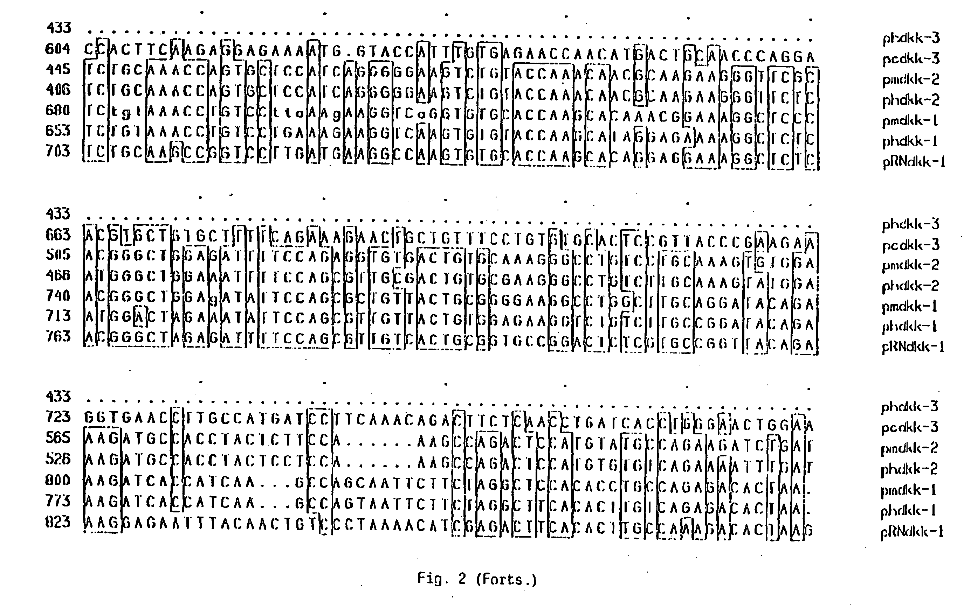

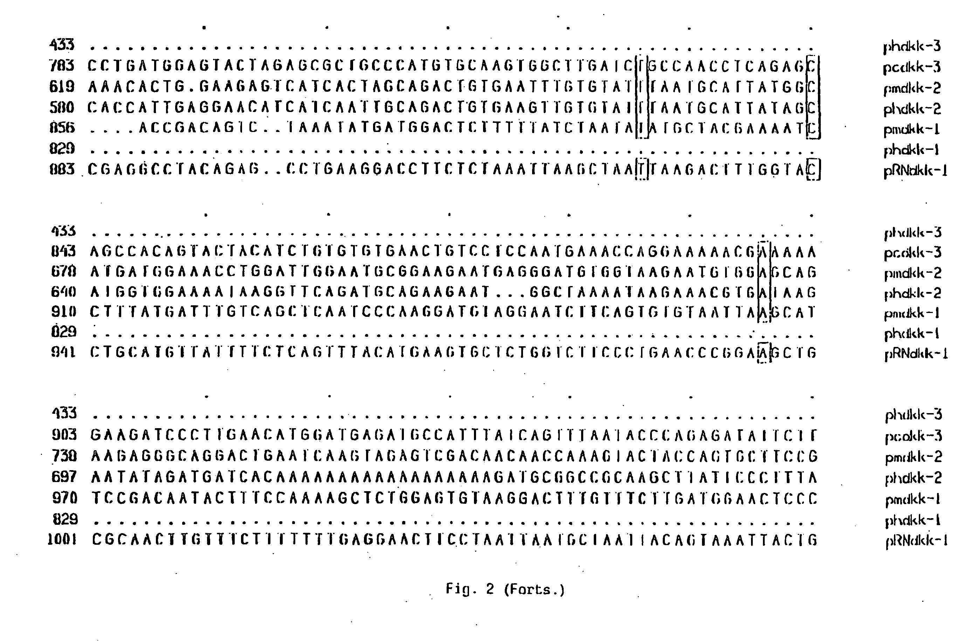

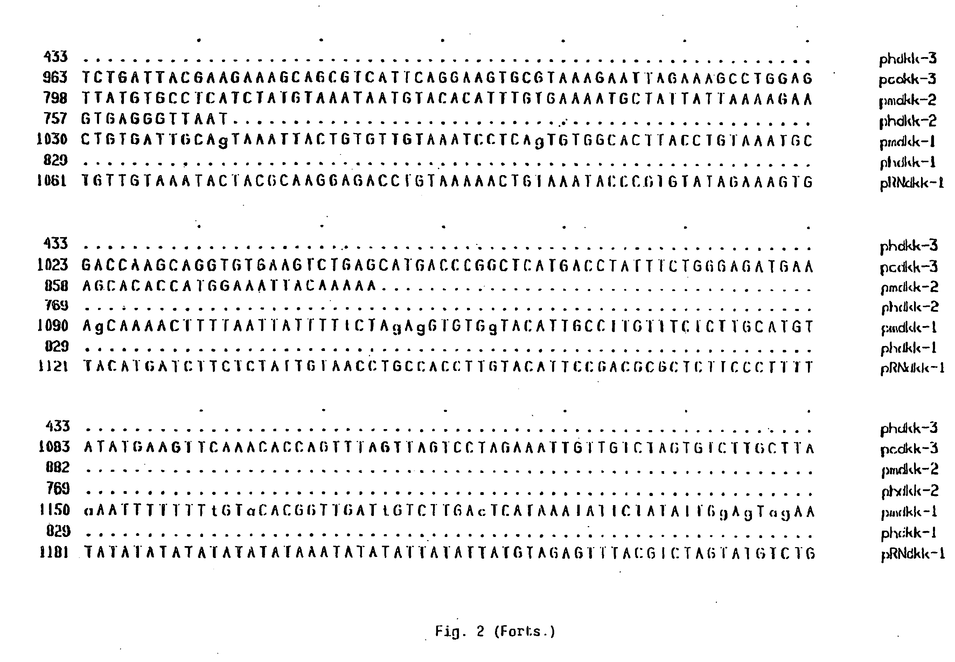

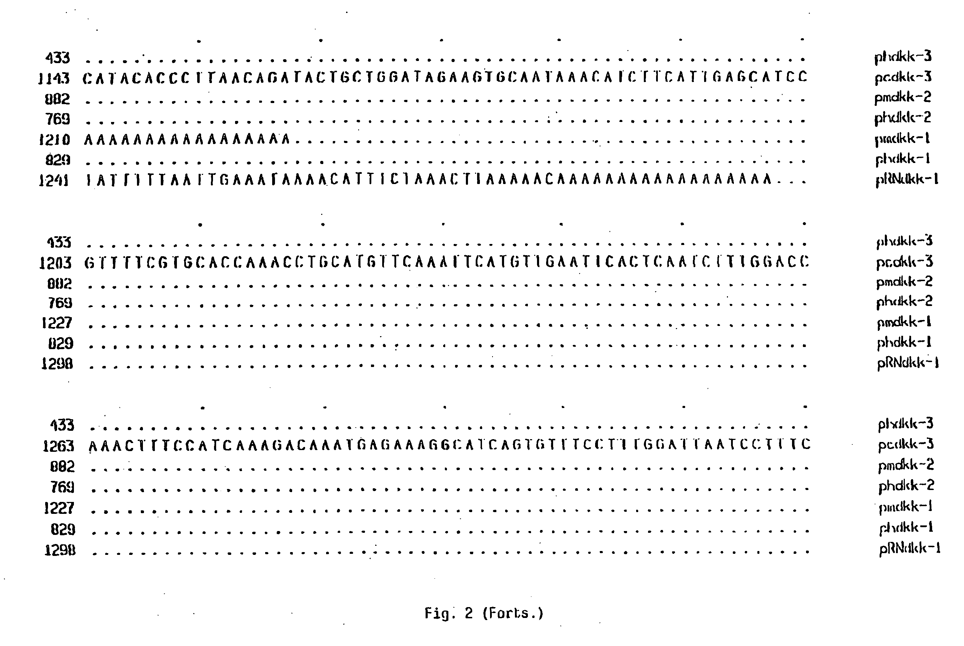

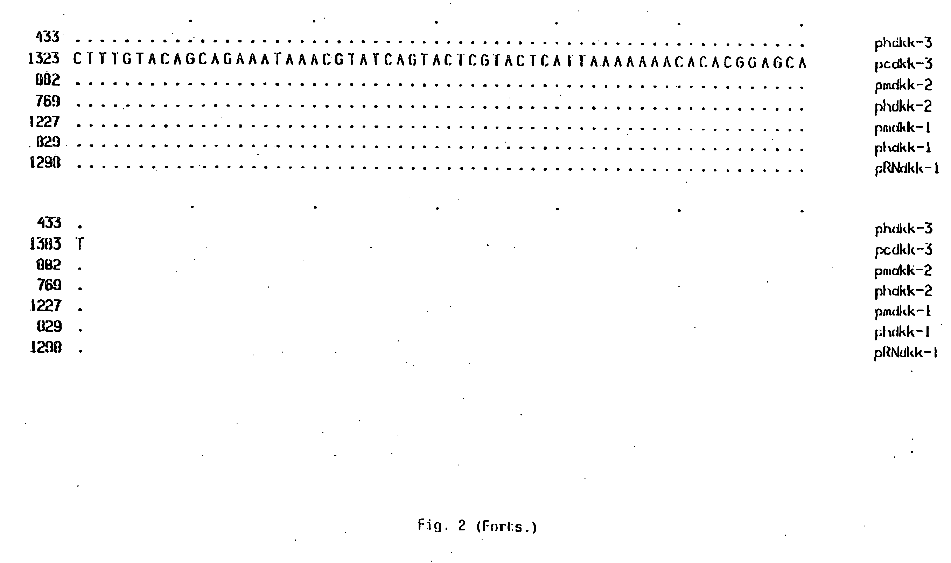

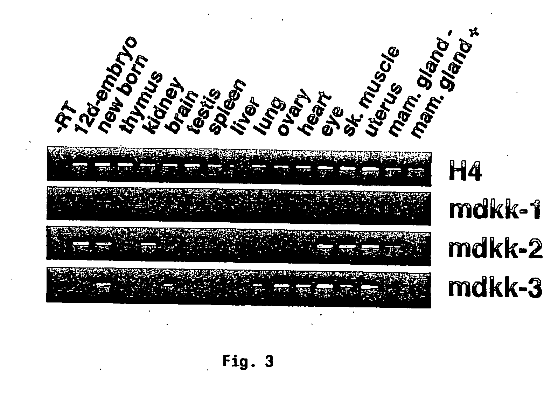

Expression des wnt-Gens, Xwnt-8, in Xenopus laevis zur Ausbildung von Siamesischen Zwillingen führt. Diese Mißbildung wird verhindert, wenn gleichzeitig das vorstehende Protein exprimiert wird. Dieses Protein ist ein sekretorisches Protein von etwa 40 kD. Varianten des Proteins sind in Form ihrer DNAs in Fig. 2 angegeben. Desweiteren hat der Anmelder erkannt, daß Varianten des Proteins in unterschiedlichen Geweben exprimiert werden (vgl. Tabelle 1 und Fig. 3).

In der vorliegenden Erfindung wird vorstehendes Protein mit "wnt-Inhibitor" (wnt-I) bezeichnet.

Ein weiterer Gegenstand der vorliegenden Erfindung ist eine für (wnt-I) kodierende DNA. Diese kann z.B. eine genomische DNA oder eine cDNA sein. Diese ist eine DNA, die folgendes umfaßt:
(a) die DNA von Fig. 2.3, 2.4, 2.5, 2.6, oder 2.7
(b) eine mit der DNA von (a) über den degenerierten genetischen Code verwandte DNA.

Die DNA von Fig. 2 umfaßt sieben DNAs, die aus Xenopus laevis, Maus, Mensch oder Huhn stammen und für (wnt-I) kodieren. Sechs dieser DNAs wurden bei der DSMZ (Deutsche Sammlung von Mikroorganismen und Zellkulturen) am 19. Sept. 1997 wie folgt hinterlegt:
- Fig. 2.1: (DNA aus Mensch) als phdkk-3 unter DSM 11762
- Fig. 2.2: (DNA aus Huhn) trägt die Bezeichnung pcdkk-3
- Fig. 2.3: (DNA aus Maus) als pmdkk-2 unter DSM 11759
- Fig. 2.4: (DNA aus Mensch) als phdkk-2 unter DSM 11761
- Fig. 2.5: (DNA aus Maus) als pmdkk-1 unter DMS 11758
- Fig. 2.6: (DNA aus Mensch) als phdkk-1 unter DSM 11760
- Fig. 2.7: (DNA aus Xenopus laevis) als pRNdkk-1 unter DSM 11757

Nachstehend wird eine erfindungsgemäße DNA in Form einer cDNA beschrieben. Diese steht beispielhaft für jede unter die vorliegende Erfindung fallende DNA.

Zur Herstellung einer erfindungsgemäßen cDNA ist es günstig, von einer Xenopus laevis-cDNA-Bibliothek auszugehen (vgl. Glinka, A. et al., Mechanisms Develope.60, (1996), 221-231). Von den einzelnen cDNA-Kionen werden mittels RNA-Polymerase entsprechende mRNAs synthetisiert. Diese werden zusammen mit mRNA von wnt-Genen, z.B. Xwnt-8, in Xenopus laevis mikroinjiziert. Es wird auf die Ausbildung von Siamesischen Zwillingen bei Xenopus laevis gescreent. Diese werden erhalten, wenn die mRNA des wnt-Gens alleine oder zusammen mit solcher Xenopus laevis mRNA mikroinjiziert wird, die nicht für (wnt-I) kodiert. Das Nicht-Auftreten von Siamesischen Zwillingen wird somit als Nachweis für das Vorliegen einer mRNA gewertet, die für (wnt-I) kodiert. Solch eine mRNA läßt unmittelbar die entsprechende cDNA erkennen.

Eine erfindunggemäße cDNA kann in einem Vektor bzw. Expressionsvektor vorliegen. Beispiele solcher sind dem Fachmann bekannt. Im Falle eines Expressionsvektors für E. coli sind dies z.B. pGEMEX, pUC-Derivate, pGEX-2T, pET3b und pQE-8. Für die Expression in Hefe sind z.B. pY100 und Ycpad1 zu nennen, während für die Expression in tierischen Zellen z.B. pKCR, pEFBOS, cDM8 und pCEV4, anzugeben sind. Für die Expression in Insektenzellen eignet sich besonders der Baculovirus-Expressionsvektor pAcSGHisNT-A.

Der Fachmann kennt geeignete Zellen, um eine, erfindungsgemäße, in einem Expressionsvektor vorliegende cDNA zu exprimieren. Beispiele solcher Zellen umfassen die E.coli-Stämme HB101, DH1, x1776, JM101, JM 109, BL21 und SG 13009, den Hefe-Stamm Saccharomyces cerevisiae und die tierischen Zellen L, 3T3, FM3A, CHO, COS, Vero und HeLa sowie die Insektenzellen sf9.

Der Fachmann weiß, in welcher Weise eine erfindungsgemäße cDNA in einen Expressionsvektor inseriert werden muß. Ihm ist auch bekannt, daß diese cDNA in Verbindung mit einer für ein anderes Protein bzw. Peptid kodierenden DNA inseriert werden kann, so daß die erfindungsgemäße cDNA in Form eines Fusionsproteins exprimiert werden kann.

Des weiteren kennt der Fachmann Bedingungen, transformierte bzw. transfizierte Zellen zu kultivieren. Auch sind ihm Verfahren bekannt, das durch die erfindungsgemäße cDNA exprimierte Protein zu isolieren und zu reinigen. Ein solches Protein, ist somit ebenfalls Gegenstand der vorliegenden Erfindung.

Ein weiterer Gegenstand der vorliegenden Erfindung ist ein gegen ein vorstehendes Protein gerichteter Antikörper. Ein solcher Antikörper kann durch übliche Verfahren hergestellt werden. Er kann monoklonal sein. Zu seiner Herstellung ist es günstig, Tiere, insbesondere Mäuse für einen monoklonalen Antikörper, mit einem vorstehenden (Fusions)protein oder Fragmenten davon zu immunisieren. Weitere "Booster" der Tiere können mit dem gleichen (Fusions)protein oder Fragmenten davon erfolgen. Der polyklonale Antikörper kann dann aus dem Serum bzw. Eigelb der Tiere erhalten werden. Für den monoklonalen Antikörper werden Milzzellen der Tiere mit Myelomzellen fusioniert.

Die vorliegende Erfindung ermöglicht es, den wnt-Signalweg besser zu untersuchen und zu verstehen. Mit einem erfindungsgemäßen Antikörper kann (wnt-I) in Organismen nachgewiesen werden. Ferner kann mit einem erfindungsgemäßen (wnt-I) ein gegen dieses Protein gerichteter Autoantikörper nachgewiesen werden. Beide Nachweise können durch übliche Verfahren, insbesondere einen Western Blot, einen ELISA, eine Immunpräzipitation oder durch Immunfluoreszenz, erfolgen. Desweiteren kann mit einer erfindungsgemäßen Nukleinsäure, insbesondere einer DNA und hiervon abgeleiteten Primern, die Expression des für (wnt-I) kodierenden Gens nachgewiesen werden. Dieser Nachweis kann in üblicher Weise, insbesondere in einem Southern Blot, erfolgen.

Somit können mit der vorliegenden Erfindung auch Prozesse besser untersucht, d.h. diagnostiziert, und verstanden werden, die mit dem wnt-Signalweg zusammenhängen. Dies sind z.B. Zellproliferation und -Differenzierung sowie Erkrankungen verschiedenster Art. Beispiele von letzteren sind Erkrankungen des Auges und der Knochen sowie Tumorerkrankungen, insbesondere Colon- und Mammakarzinom sowie Melanom.

Desweiteren eignet sich die vorliegende Erfindung, Maßnahmen für und gegen das Vorliegen von (wnt-I) in Organismen zu ergreifen. Mit einem erfindungsgemäßen Antikörper kann (wnt-I) in Organismen inhibiert werden. Andererseits kann mit einem erfindungsgemäßen (wnt-I), insbesondere nach Kopplung an ein vom Körper nicht als fremd angesehenes Protein, z.B. Transferrin oder BSA, die Menge von (wnt-I) in Organismen erhöht werden. Entsprechendes kann auch mit einer erfindungsgemäßen Nukleinsäure, insbesondere einer DNA, erreicht werden, die unter die Kontrolle eines in bestimmten Geweben induzierbaren Promotors gestellt wird und nach ihrer Expression zur Bereitstellung von (wnt-I) in diesen Geweben führt. Darüberhinaus kann eine erfindungsgemäße Nukleinsäure, insbesondere eine DNA, auch zur Inhibierung von (wnt-I) genutzt werden. Hierzu wird die Nukleinsäure, z.B. als Basis für die Erstellung von Anti-Sinn-Oligonukleotiden zur Expressions-Inhibierung des für (wnt-I) kodierenden Gens verwendet.

Somit stellt die vorliegende Erfindung auch die Möglichkeit bereit, in den wnt-Signalweg aktivierend bzw. inhibierend einzugreifen. Erstes könnte z.B durch Verabreichung eines erfindungsgemäßen Antikörpers gegen (wnt-I) erfolgen. Für letzteres bietet sich an, erfindungsgemäßes (wnt-I) zu verabreichen. Die Aktivierung des wnt-Signalwegs könnte sinnvoll sein, wenn daran gedacht wird, Organismen für Organspende zu züchten. Die Inhibierung des wnt-Signalwegs bietet sich allerdings an, um therapeutisch bei Erkrankungen von Knochen und des Auges sowie bei Tumorerkrankungen, insbesondere Colon- und Mammakarzinomen sowie Melanom, eingreifen zu können.

Insbesondere zeichnet sich die vorliegende Erfindung dadurch aus, daß sie gewebespezifisch eingesetzt werden kann. Dies gilt sowohl für Diagnose als auch für Therapie. Beispielsweise eignet sich eine erfindungsgemäße DNA, DKK-1, ein entsprechendes Protein bzw. ein Antikörper davon besonders für Gewebe, wie Gehirn, Herz, Gefäße, Knochen, Knorpel, Bindegewebe und Auge. Ferner eignet sich eine erfindungsgemäße DNA, DKK-2, ein entsprechendes Protein bzw. ein Antikörper davon besonders für Gewebe, wie Gehirn, Herz, Gefäße, Knochen, Bindegewebe, Nieren, Hoden, Milz, Ovarien, Muskel, Uterus, Knorpel, Auge und Brustdrüse.

### Kurze Beschreibung der Zeichnungen:

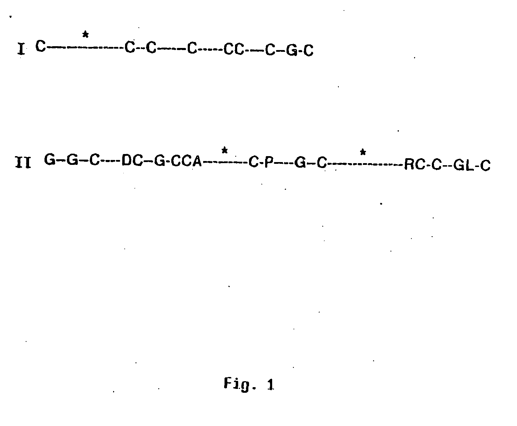

- Fig. 1: zeigt die Aminosäure-Konsensus-Sequenzen I und II eines erfindungsgemäßen (wnt-I). Die Angabe "-" bedeutet eine Aminosäure, wobei die Zahl der Aminosäuren variabel ist, wenn sie einen Stern aufweisen,
- Fig. 2: zeigt die Basensequenz von sieben (wnt-I) kodierenden DNAs mit Angabe der Basen, die zu den Aminosäure-Konsensus-Sequenzen von (wnt-I) beitragen.
- Fig. 3: zeigt die Expression von drei (wnt-I) kodierenden DNAs, DKK-1, DKK-2 und DKK-3, in Geweben.

Die vorliegende Erfindung wird durch die nachstehenden Beispiele erläutert.

### Beispiel 1: Herstellung und Reinigung eines (wnt-I)

Zur Herstellung eines (wtn-I) wurde die DNA von Fig. 2.6, phdkk-1 mit Bam HI-Linkern versehen, anschließend mit Bam HI gespalten und in den mit Bam HI gespaltenen Expressionsvektors pQE-8 (Qiagen) inseriert. Es wurde das Expressionsplasmid pQ/wnt-I erhalten. Ein solches kodiert für ein Fusionsprotein aus 6 Histidin-Resten (N-Terminuspartner) und einem erfindungsgemäßen (wnt-I) (C-Terminuspartner). pQ/wnt-I wurde zur Transformation von E.coli SG 13009(vgl. Gottesman, S. et al., J. Bacteriol. 148, (1981), 265-273) verwendet. Die Bakterien wurden in einem LB-Medium mit 100µg/ml Ampicillin und 25µg/ml Kanamycin kultiviert und 4 h mit 60µM Isopropyl-β-D-Thiogalactopyranosid (IPTG) induziert. Durch Zugabe von 6 M Guanidinhydrochlorid wurde eine Lyse der Bakterien erreicht, anschließend wurde mit dem Lysat eine Chromatographie (Ni-NTA-Resin) in Gegenwart von 8 M Harnstoff entsprechend der Angaben des Herstellers (Diagen) des Chromatographie-Materials durchgeführt. Das gebundene Fusionsprotein wurde in einem Puffer mit pH 3,5 eluiert. Nach seiner Neutralisierung wurde das Fusionsprotein einer 18 % SDS-Polyacrylamid-Gelelektrophorese unterworfen und mit Coomassie-Blau angefärbt (vgl. Thomas, J.O. und Kornberg, R.D., J.Mol.Biol. 149 (1975), 709-733).

Es zeigte sich, daß ein (Fusions)protein in hochreiner Form hergestellt werden kann.

### Beispiel 2: Herstellung und Nachweis eines Antikörpers

Ein Fusionsprotein von Beispiel 1 wurde einer 18 % SDS-Polyacrylamid-Gelelektrophorese unterzogen. Nach Anfärbung des Gels mit 4 M Natriumacetat wurde eine ca. 40 kD Bande aus dem Gel herausgeschnitten und in Phosphat gepufferter Kochsalzlösung inkubiert. Gel-Stücke wurden sedimentiert, bevor die Proteinkonzentration des Überstandes durch eine SDS-Polyacrylamid-Gelelektrophorese, der eine Coomassie-Blau-Färbung folgte, bestimmt wurde. Mit dem Gel-gereinigten Fusionsprotein wurden Tiere wie folgt immunisiert:

### Immunisierungsprotokoll für polyklonale Antikörper im Kaninchen

Pro Immunisierung wurden 35µg Gel-gereinigtes Fusionsprotein in 0,7 ml PBS und 0,7 ml komplettem bzw. inkomplettem Freund's Adjuvans eingesetzt.
- Tag O:: 1. Immunisierung (komplettes Freund's Adjuvans)
- Tag 14:: 2. Immunisierung (inkomplettes Freund's Adjuvans; icFA)
- Tag 28:: 3. Immunisierung (icFA)
- Tag 56:: 4. Immunisierung (icFA)
- Tag 80:: Ausbluten

Das Serum des Kaninchens wurde im Immunoblot getestet. Hierzu wurde ein Fusionsprotein von Beispiel 1 einer SDS-Polyacrylamid-Gelelektrophorese unterzogen und auf ein Nitrocellulosefilter übertragen (vgl. Khyse-Andersen, J., J. Biochem. Biophys. Meth. 10, (1984), 203-209). Die Western Blot-Analyse wurde wie in Bock, C.-T. et al., Virus Genes 8, (1994), 215-229, beschrieben, durchgeführt. Hierzu wurde das Nitrocellulosefilter eine Stunde bei 37°C mit einem ersten Antikörper inkubiert. Dieser Antikörper war das Serum des Kaninchens (1:10000 in PBS). Nach mehreren Waschschritten mit PBS wurde das Nitrocellulosefilter mit einem zweiten Antikörper inkubiert. Dieser Antikörper war ein mit alkalischer Phosphatase gekoppelter monoklonaler Ziege Anti-Kaninchen-lgG-Antikörper (Dianova) (1:5000) in PBS. Nach 30-minütiger Inkubation bei 37°C folgten mehrere Waschschritte mit PBS und anschließend die alkalische Phosphatase-Nachweisreaktion mit Entwicklerlösung (36µM 5' Bromo-4-chloro-3-indolylphosphat, 400µM Nitroblau-tetrazolium, 100mM Tris-HCl, pH 9.5, 100 mM NaCl, 5 mM MgCl₂) bei Raumtemperatur, bis Banden sichtbar waren.

Es zeigte sich, daß, polyklonale Antikörper hergestellt werden können.

### lmmunisierungsprotokoll für polyklonale Antikörper im Huhn

Pro Immunisierung wurden 40µg Gel-gereinigtes Fusionsprotein in 0,8 ml PBS und 0,8 ml komplettem bzw. inkomplettem Freund's Adjuvans eingesetzt.
- Tag O.: 1. Immunisierung (komplettes Freund's Adjuvans)
- Tag 28:: 2. Immunisierung (inkomplettes Freund's Adjuvans; icFA)
- Tag 50:: 3. Immunisierung (icFA)

Aus Eigelb wurden Antikörper extrahiert und im Western Blot getestet. Es wurden, polyklonale Antikörper nachgewiesen.

### Immunisierungsprotokoll für monoklonale Antikörper der Maus

Pro Immunisierung wurden 12µg Gel-gereinigtes Fusionsprotein in 0,25 ml PBS und 0,25 ml komplettem bzw. inkomplettem Freund's Adjuvans eingesetzt; bei der 4. Immunisierung war das Fusionsprotein in 0,5 ml (ohne Adjuvans) gelöst.
- Tag O.: 1. Immunisierung (komplettes Freund's Adjuvans)
- Tag 28:: 2. Immunisierung (inkomplettes Freund's Adjuvans; icFA)
- Tag 56:: 3. Immunisierung (icFA)
- Tag 84:: 4. Immunisierung (PBS)
- Tag 87:: Fusion

Überstände von Hybridomen wurden im Western Blot getestet. Erfindungsgemäße, monoklonale Antikörper wurden nachgewiesen.

**Tabelle 1: Expression von DNAs in Mausembryonen**

| | **Dkk-1** | **Dkk-2** | **Dkk-3** |
|---|---|---|---|
| **Neuroepithelium** | | | |
| | | | |
| E9.5 diencephalon | +++ ventral | +++ medial | + medial |
| | | | |
| E 12.5 | telencephalon M/mantle | hypothalamus | telencephalon M/ ventricular zone |
| | | | |
| Eye | pigmented epithelium | choroid | retina |
| | | | |
| Spinal cord | -/+ | - | ventricular zone Roof plate |
| | | | |

| **Mesoderm:** | | | |
|---|---|---|---|
| | | | |
| Heart E10 | bulbis cordis Endocardium septum transversum | endothelium | myocardium |
| | | | |
| Heart E12 | endocardial cushion | endothelium | endocardial cushion |
| | | | |
| Blood vessels | +++ aorta | +++ pulmonary artery | +++ aorta + pulmonary artery |
| | | | |
| Limbbud mesenchyme | E9 S | I | D |
| | | | |
| Bone E12 | perichondrium | S/mesenchyme | perichondrium I/mesenchyme |
| | | | |
| Bone E15 | Ossitication centers | - | - |
| | | | |
| Urogenital | nephric duct S-shaped body Comma shaped body | metanephric mesenchyme | - |
| | | | |
| Palate | +++ | ++ | + |
| | | | |
| Hair follicle | +++ mesenchyme + epithelium | + - | + - |
| | | | |
| Tooth mesenchyme | - | - | +++ |
| | | | |
| Trunk mesoderm | +/- | +++ | ++ |

| | | | |
|---|---|---|---|
| Legende: Mesoderm: (D) decp, (I) intermediate (L) lateral, (M) medial, (S), superficial. Expressionshöhe: (-) absence, (+/-) very weak expression, (+) medium, (++) strong, (+++) very strong. | | | |

## Patentansprüche

1. Inhibitor-Protein des wnt-Signalwegs, wobei das Protein durch die DNA von Fig. 2.3, 2.4, 2.5, 2.6 oder 2.7 bzw. durch eine mit dieser DNA über den degenerierten genetischen Code verwandte DNA kodiert wird, wobei das Protein zumindest eine der Aminosäure-Konsensus-Sequenzen I und II umfasst: wobei die Angabe "-'" eine Aminosäure bedeutet, wobei die Zahl der Aminosäuren variabel ist, wenn sie einen Stern aufweisen.

2. DNA, kodierend für das Protein nach Anspruch 1, wobei die DNA jene von Fig. 2.3, 2.4, 2.5, 2.6 oder 2.7 bzw. eine mit dieser DNA über den degenerierten genetischen Code verwandte DNA ist

3. Expressionsplasmid, umfassend die DNA nach Anspruch 2.

4. Transformante, enthaltend das Expressionsplasmid nach Anspruch 3.

5. Verfahren zur Herstellung des Proteins nach Anspruch 1, umfassend die Kultivierung der Transformante nach Anspruch 4 unter geeigneten Bedingungen.

6. Monoklonaler Anikörper, gerichtet gegen das Protein nach Anspruch 1.

## Claims

1. An inhibitor protein of the wnt signal path, wherein the protein is encoded by the DNA of figures 2.3, 2.4, 2.5, 2.6 or 2.7 and by a DNA related to this DNA via the degenerated genetic code, respectively, wherein the protein comprises at least one of the amino acid consensus sequences I and II: wherein the indication "-" designates an amino acid, wherein the number of amino acids is variable when they have an asterisk.

2. A DNA coding for the protein according to claim 1, wherein the DNA is that of figure 2.3, 2.4, 2.5, 2.6 or 2.7 and/or a DNA related to this DNA via the degenerated genetic code.

3. An expression plasmid, comprising the DNA according to claim 2.

4. A transformant, containing the DNA according to claim 3.

5. A method of producing the protein according to claim 1, comprising culturing the transformant according to claim 4 under suitable conditions.

6. A monoclonal antibody directed against the protein according to claim 1.

## Revendications

1. Protéine inhibitrice de la voie de signalisation wnt, dans laquelle la protéine est codée par l'ADN des figures 2.3, 2.4, 2.5, 2.6, ou 2.7 ou bien par un ADN utilisé avec cet ADN par l'intermédiaire d'un code génétique dégénéré, la protéine comprenant au moins l'une des séquences de consensus d'acides aminés I et II : l'indication "-" signifiant au moins un acide aminé, le nombre d'acides aminés étant variable lorsqu'ils présentent un astérisque.

2. ADN de codage de la protéine selon la revendication 1, **caractérisé en ce que** l'ADN est l'un des ADN suivant les figures 2.3, 2.4, 2.5, 2.6, ou 2.7 ou bien un ADN utilisé avec cet ADN par l'intermédiaire d'un code génétique dégénéré.

3. Plasmide d'expression comprenant l'ADN selon la revendication 2.

4. Produit de transformation renfermant le plasmide d'expression selon la revendication 3.

5. Procédé d'obtention de la protéine selon la revendication 1, comprenant la culture du produit de transformation selon la revendication 4 dans des conditions appropriées.

6. Anticorps monoclonal dirigé contre la protéine selon la revendication 1.


### SEQUENZPROTOKOLL

(1) ALLGEMEINE ANGABEN:
   (i) ANMELDER:
      (A) NAME: Deutsches Krebsforschungszentrum
      (B) STRASSE: Im Neuenheimer Feld 280
      (C) ORT: Heidelberg
      (E) LAND: Deutschland
      (F) POSTLEITZAHL: 69120
   (ii) BEZEICHNUNG DER ERFINDUNG: Inhibitorprotein des wnt-Signalwegs
   (iii) ANZAHL DER SEQUENZEN: 7
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30(EPA)
   (v) DATEN DER VORANMELDUNG:
      ANMELDENUMMER: DE 19747418.7
(2) ANGABEN ZU SEQ ID NO: 1:
   (i) SEQUENZKENNZEICHEN:
      (A) LÄNGE: 1297 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (xi) SEQUENZBESCHREIBUNG: SEQ ID NO: 1:
   GACAGTCGGA GCCGGCGCTG CAGCATCAAA GGGACTTATC TTGGAGGACT TGTGAATTCT     60
   CATCCTGCCA TTGTGGTTAC TGAGTCTGGT TGGACAGAGG AATGGGCAGC AACATGTTCC    120
   CGGTGCCTCT TATTGTCTTT TGGGGTTTTA TCTTGGATGG GGCACTTGGC TTTGTCATGA    180
   TGACCAACTC CAACTCCATC AAGAATGTGC CGGCGGCACC AGCAGGTCAG CCCATTGGCT    240
   ACTACCCTGT GAGCGTCAGT CCGGACTCCC TATATGATAT TGCCAACAAG TACCAACCTC    300
   TGGATGCCTA CCCGCTCTAC AGTTGCACGG AAGATGATGA CTGTGCCCTT GATGAATTCT    360
   GTCACAGTTC CAGAAACGGC AACTCTCTGG TTTGCTTGGC ATGCCGGAAA CGCAGAAAGC    420
   GTTGCCTGAG GGACGCCATG TGCTGCACAG GCAACTACTG TAGCAACGGA ATTTGTGTCC    480
   CTGTGGAGCA AGATCAAGAG CGCTTCCAAC ACCAGGGATA CCTGGAAGAA ACCATTCTGG    540
   AAAACTATAA TAATGCTGAT CATGCAACAA TGGATACTCA TTCCAAATTA ACCACGTCCC    600
   CATCTGGAAT GCAGCCCTTT AAAGGCCGTG ATGGTGATGT TTGCCTCCGA TCAACTGACT    660
   GTGCGCCAGG TCTATGCTGT GCCCGTCATT TCTGGTCAAA GATCTGCAAG CCGGTCCTTG    720
   ATGAAGGCCA AGTGTGCACC AAGCACAGGA GGAAAGGCTC TCACGGGCTA GAGATTTTCC    780
   AGCGTTGTCA CTGCGGTGCC GGACTCTCGT GCCGGTTACA GAAAGGAGAA TTTACAACTG    840
   TCCCTAAAAC ATCGAGACTT CACACTTGCC AAAGACACTA AGCGAGGCCT ACAGAGCCTG    900
   AAGGACCTTC TCTAAATTAA GCTAATTAAG ACTTTGGTAC CTGCATGTTA TTTTCTCAGT    960
   TTACATGAAG TGCTCTGGTC TTCCCTGAAC CCGGAAGCTG CGCAACTTGT TTCTTTTTTT   1020
   GAGGAACTTC CTAATTAATG CTAATTACAG TAAATTACTG TGTTGTAAAT ACTACGCAAG   1080
   GAGACCTGTA AAAACTGTAA ATACCCGTGT ATAGAAAGTG TACATGATCT TCTCTATTGT   1140
   AACCTGCCAC CTTGTACATT CCGACGCGCT CTTCCCTTTT TATATATATA TATATATAAA   1200
   TATATATTAT ATTATGTAGA GTTTACGTCT AGTATGTCTG TATTTTTAAT TGAAATAAAA   1260
   CATTTCTAAA CTTAAAAACA AAAAAAAAAA AAAAAAA                             1297
(2) ANGABEN ZU SEQ ID NO: 2:
   (i) SEQUENZKENNZEICHEN:
      (A) LÄNGE: 881 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (xi) SEQUENZBESCHREIBUNG: SEQ ID NO: 2:
   TGCAGGCATG AACAAGGACT GGGTTCGGCG GCAGTGAGAA GGGCAAAAGC CTGGGGCAGG    60
   CCTACCCTTG CAGCAGTGAT AAGGAATGTG AAGTTGGAAG ATACTGCCAC AGTCCCCACC   120
   AAGGTTCATC AGCCTGCATG CTCTGTAGGA GGAAAAAGAA ACGATGCCAC AGAGATGGGA   180
   TGTGTTGCCC TGGTACCCGC TGCAATAATG GAATCTGCAT CCCAGTCACT GAGAGCATCC   240
   TCACCCCACA TATCCCAGCT CTGGATGGCA CCCGGCATAG AGATCGCAAC CATGGTCACT   300
   ATTCCAACCA TGACCTGGGA TGGCAGAATC TAGGAAGGCC ACACTCCAAG ATGCCTCATA   360
   TAAAAGGACA TGAAGGAGAC CCATGCCTAC GGTCATCAGA CTGCATTGAT GGGTTTTGTT   420
   GTGCTCGCCA CTTCTGGACC AAAATCTGCA AACCAGTGCT CCATCAGGGG GAAGTCTGTA   480
   CCAAACAACG CAAGAAGGGT TCGCACGGGC TGGAGATTTT CCAGAGGTGT GACTGTGCAA   540
   AGGGCCTGTC CTGCAAAGTG TGGAAAGATG CCACCTACTC TTCCAAAGCC AGACTCCATG   600
   TATGCCAGAA GATCTGATAA ACACTGGAAG AGTCATCACT AGCAGACTGT GAATTTGTGT   660
   ATTTAATGCA TTATGGCATG ATGGAAACCT GGATTGGAAT GCGGAAGAAT GAGGGATGTG   720
   GTAAGAATGT GGAGCAGAAG AGGGCAGGAC TGAATCAAGT AGAGTCGACA ACAACCAAAG   780
   TACTACCAGT GCTTCCGTTA TGTGCCTCAT CTATGTAAAT AATGTACACA TTTGTGAAAA   840
   TGCTATTATT AAAAGAAAGC ACACCATGGA AATTACAAAA A                       881
(2) ANGABEN ZU SEQ ID NO: 3:
   (i) SEQUENZKENNZEICHEN:
      (A) LÄNGE: 1226 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (xi) SEQUENZBESCHREIBUNG: SEQ ID NO: 3:
   GACCCACGCG TCCGTGCCTG TTTGCGTCCT TCGGAGATGA TGGTTGTGTG TGCACCGGCA     60
   GCTGTCCGGT TCTTGGCCGT GTTTACAATG ATGGCTCTCT GCAGCCTCCC TCTGCTAGGA    120
   GCCAGTGCCA CCTTGAACTC AGTTCTCATC AATTCCAACG CGATCAAGAA CCTGCCCCCA    180
   CCGCTGGGTG GTGCTGGGGG GCAGCCGGGC TCTGCTGTCA GTGTGGCGCC GGGAGTTCTC    240
   TATGAGGGCG GGAACAAGTA CCAGACTCTT GACAACTACC AGCCCTACCC TTGCGCTGAA    300
   GATGAGGAGT GCGGCTCTGA CGAGTACTGC TCCAGCCCCA GCCGCGGGGC AGCCGGCGTC    360
   GGAGGTGTAC AGATCTGTCT GGCTTGCCGA AAGCGCAGGA AGCGCTGCAT GACGCACGCT    420
   ATGTGCTGCC CCGGGAACTA CTGCAAAAAT GGAATATGCA TGCCCTCTGA CCACAGCCAT    480
   TTTCCTCGAG GGGAAATTGA GGAAAGCATC ATTGAAAACC TTGGTAATGA CCACAACGCC    540
   GCCGCGGGGG ATGGATATCC CAGAAGAACC ACACTGACTT CAAAAATATA TCACACCAAA    600
   GGACAAGAAG GCTCCGTCTG CCTCCGATCA TCAGACTGTG CCGCAGGGCT GTGTTGTGCA    660
   AGACACTTCT GGTCCAAGAT CTGTAAACCT GTCCTTAAAG AAGGTCAGGT GTGCACCAAG    720
   CACAAACGGA AAGGCTCCCA CGGGCTGGAG ATATTCCAGC GCTGTTACTG CGGGGAAGGC    780
   CTGGCTTGCA GGATACAGAA AGATCACCAT CAAGCCAGCA ATTCTTCTAG GCTCCACACC    840
   TGCCAGAGAC ACTAAACCGA CAGTCTAAAT ATGATGGACT CTTTTTATCT AATATATGCT    900
   ACGAAAATCC TTTATGATTT GTCAGCTCAA TCCCAAGGAT GTAGGAATCT TCAGTGTGTA    960
   ATTAAGCATT CCGACAATAC TTTCCAAAAG CTCTGGAGTG TAAGGACTTT GTTTCTTGAT   1020
   GGAACTCCCC TGTGATTGCA GTAAATTACT GTGTTGTAAA TCCTCAGTGT GGCACTTACC   1080
   TGTAAATGCA GCAAAACTTT TAATTATTTT TCTAGAGGTG TGGTACATTG CCTTGTTTCT   1140
   CTTGCATGTA AATTTTTTTT GTACACGGTT GATTGTCTTG ACTCATAAAT ATTCTATATT   1200
   GGAGTAGAAA AAAAAAAAAA AAAAAA                                        1226
(2) ANGABEN ZU SEQ ID NO: 4:
   (i) SEQUENZKENNZEICHEN:
      (A) LÄNGE: 768 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (xi) SEQUENZBESCHREIBUNG: SEQ ID NO: 4:
   ATACGACTCA CTATAGGGAA TTTGGCCCTC GAGGCCAAGA ATTCGGCACG AGGGTTGGGA    60
   GGTATTGCCA CAGTCCCCAC CAAGGATCAT CGGCCTGCAT GGTGTGTCGG AGAAAAAAGA   120
   AGCGCTGCCA CCGAGATGGC ATGTGCTGCC CCAGTACCCG CTGCAATAAT GGCATCTGTA   180
   TCCCAGTTAC TGAAAGCATC TTAACCCCTC ACATCCCGGC TCTGGATGGT ACTCGGCACA   240
   GAGATCGAAA CCACGGTCAT TACTCAAACC ATGACTTGGG ATGGCAGAAT CTAGGAAGAC   300
   CACACACTAA GATGTCACAT ATAAAAGGGC ATGAAGGAGA CCCCTGCCTA CGATCATCAG   360
   ACTGCATTGA AGGGTTTTGC TGTGCTCGTC ATTTCTGGAC CAAAATCTGC AAACCAGTGC   420
   TCCATCAGGG GGAAGTCTGT ACCAAACAAC GCAAGAAGGG TTCTCATGGG CTGGAAATTT   480
   TCCAGCGTTG CGACTGTGCG AAGGGCCTGT CTTGCAAAGT ATGGAAAGAT GCCACCTACT   540
   CCTCCAAAGC CAGACTCCAT GTGTGTCAGA AAATTTGATC ACCATTGAGG AACATCATCA   600
   ATTGCAGACT GTGAAGTTGT GTATTTAATG CATTATAGCA TGGTGGAAAA TAAGGTTCAG   660
   ATGCAGAAGA ATGGCTAAAA TAAGAAACGT GATAAGAATA TAGATGATCA CAAAAAAAAA   720
   AAAAAAAAAG ATGCGGCCGC AAGCTTATTC CCTTTAGTGA GGGTTAAT                768
(2) ANGABEN ZU SEQ ID NO: 5:
   (i) SEQUENZKENNZEICHEN:
      (A) LÄNGE: 828 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (xi) SEQUENZBESCHREIBUNG: SEQ ID NO: 5:
   TGGCCCCGCA CGCCAAAAAT TCGGCACGAG GGTCTGGCAC TCAGAGGATG CTCTGACCTT    60
   GAAAGGGTCC TATCTGGAGA CGAGGGAGTA CAACGTGCTG AATGTGTGCG GTTCAGGGAG   120
   CATTTGGTAA CCCTGCATTT GGGAGCAGTG GGCACTAACC GGTTTTGGAG AGGTGGACAC   180
   ATAAGGACTG TGATCAGCGC CCGGGTCCAA GAGGGCGGGT ACCTGGACCT CTGGGTGCCT   240
   CACCCTCTCC CCGAACCCTT CCCACAGCCG TACCCGTGCG CAGAGGACGA GGAGTGCGGC   300
   ACTGATGAGT ACTGCGCTAG TCCCACCCCG CGGAGGGGAC CGCCGGCCGT GCAAATCTGT   360
   CTCGCCTGCA GGAAGCGCCG AAAACGCTGC ATGCGTCACG CTATGTGCTG CCCCGGGAAT   420
   TACTGCAAAA ATGGAATATG TGTGTCTTCT GATCAAAATC ATTTCCGAGG AGAAATTGAG   480
   GAAACCATCA CTGAAAGCTT TGGTAATGAT CATAGCACCT TGGATGGGTA TTCCAGAAGA   540
   ACCACCTTGT CTTCAAAAAT GTATCACACC AAAGGACAAG AAGGTTCTGT TTGTCTCCGG   600
   TCATCAGACT GTGCCTCAGG ATTGTGTTGT GCTAGACACT TCTGGTCCAA GATCTGTAAA   660
   CCTGTCCTGA AAGAAGGTCA AGTGTGTACC AAGCATAGGA GAAAAGGCTC TCATGGACTA   720
   GAAATATTCC AGCGTTGTTA CTGTGGAGAA GGTCTGTCTT GCCGGATACA GAAAGATCAC   780
   CATCAAGCCA GTAATTCTTC TAGGCTTCAC ACTTGTCAGA GACACTAA               828
(2) ANGABEN ZU SEQ ID NO: 6:
   (i) SEQUENZKENNZEICHEN:
      (A) LÄNGE: 432 Basenpaare
      (B) ART: Nucleotid
      (C) STRANG FORM: Einzelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (xi) SEQUENZBESCHREIBUNG: SEQ ID NO: 6:
   GCGGTGGCGG CCGCTCTAGA ATAGTGGATC CCCCGGGCTG CAGGAATTCG GCACGAGCGG    60
   CTGCGGGCGC AGAGCGGAGA TGCAGCGGCT TGGGCCACCC TGCTGTGCCT GCTGCTGGCG   120
   GCGGCGGTCC CCACGGCCCC CGCGCCCGCT CCGACGGCGA CCTCGGCTCC AGTCAAGCCC   180
   GGCCCGGCTC TCAGCTACCC GCAGGAGGAG GCCACCCTCA ATGAGATGTT CCGCGGGTGA   240
   GGAACTGATG GAGGACACGC AGCACAAATT GCGCAGCGCG GTGGAAGAGA TGGAGGCAGA   300
   AGAAGCTGCT GCTAAAGCAA TCATCAGAAG TGAACCTGGC AAACTTACCT CCCAGCTATC   360
   ACAATGAGAC CAACACAGAC ACGAAGGTTG GAAATAATAA CCATCCATGT GCACCGAGAA   420
   ATTCACAAGT TT                                                      432
(2) ANGABEN ZU SEQ ID NO: 7:
   (i) SEQUENZKENNZEICHEN:
      (A) LÄNGE: 1383 Basenpaare
      (B) ART: Nucleotid
      (C) STRANGFORM: Einzelstrang
      (D) TOPOLOGIE: linear
   (ii) ART DES MOLEKÜLS: Genom-DNA
   (iii) HYPOTHETISCH: NEIN
   (iv) ANTISENSE: NEIN
   (xi) SEQUENZBESCHREIBUNG: SEQ ID NO: 7:
   CGGCGAGCGG CAGCGGCGGC TGAGGAGCGC CGGGGATGCG GCGGGGAGAG GGACCGGCGC   60
   CGCGGCGGCG ATGGCTGCTG CTGTTGGCCG TGCTGGCGGC TCTGTGCTGC GCCGCGGCCG  120
   GGAGCGGCGG GCGGCGGCGA GCGGCCAGCC TGGGCGAGAT GCTGCGGGAG GTGGAGGCGC  180
   TGATGGAGGA CACGCAGCAC AAGCTGCGCA ACGCCGTGCA GGAGATGGAA GCTGAAGAAG  240
   AAGGGGCAAA AAAACTGTCA GAAGTAAACT TTGAAAACTT ACCTCCCACC TACCATAATG  300
   AGTCCAACAC AGAAACCAGA ATTGGTAATA AAACTGTTCA GACTCATCAA GAAATTGATA  360
   AGGTTACAGA TAACAGAACT GGATCAACAA TTTTTTCCGA GACAATTATT ACATCTATAA  420
   AGGGTGGAGA AAACAAAAGA AATCATGAGT GTATCATTGA TGAAGACTGT GAAACAGGAA  480
   AGTATTGCCA GTTCTCCACC TTTGAATACA AGTGTCAGCC CTGTAAAACC CAGCATACAC  540
   ACTGCTCACG AGATGTTGAA TGCTGCGGAG ACCAGCTTTG TGTTTGGGGT GAGTGCAGGA  600
   AAGCCACTTC AAGAGGAGAA AATGGTACCA TTTGTGAGAA CCAACATGAC TGCAACCCAG  660
   GAACGTGCTG TGCTTTTCAG AAAGAACTGC TGTTTCCTGT GTGCACTCCG TTACCCGAAG  720
   AAGGTGAACC TTGCCATGAT CCTTCAAACA GACTTCTCAA CCTGATCACC TGGGAACTGG  780
   AACCTGATGG AGTACTAGAG CGCTGCCCAT GTGCAAGTGG CTTGATCTGC CAACCTCAGA  840
   GCAGCCACAG TACTACATCT GTGTGTGAAC TGTCCTCCAA TGAAACCAGG AAAAACGAAA  900
   AAGAAGATCC CTTGAACATG GATGAGATGC CATTTATCAG TTTAATACCC AGAGATATTC  960
   TTTCTGATTA CGAAGAAAGC AGCGTCATTC AGGAAGTGCG TAAAGAATTA GAAAGCCTGG 1020
   AGGACCAAGC AGGTGTGAAG TCTGAGCATG ACCCGGCTCA TGACCTATTT CTGGGAGATG 1080
   AAATATGAAG TTCAAACACC AGTTTAGTTA GTCCTAGAAA TTGTTGTCTA GTGTCTTGCT 1140
   TACATACACC CTTAACAGAT ACTGCTGGAT AGAAGTGCAA TAAACATCTT CATTGAGCAT 1200
   CCGTTTTCGT GCACCAAACC TGCATGTTCA AATTCATGTT GAATTCACTC AATCTTTGGA 1260
   CCAAACTTTC CATCAAAGAC AAATGAGAAA GGCATCAGTG TTTCCTTTGG ATTAATCCTT 1320
   TCCTTTGTAC AGCAGAAATA AACGTATCAG TACTCGTACT CATTAAAAAA ACACACGGAG 1380
   CAT                                                               1383